# EUROPEAN PATENT SPECIFICATION

(11) **EP 3 701 938 B1**
(45) Date of publication and mention of the grant of the patent: **15.11.2023**
(21) Application number: 19160293.7
(22) Date of filing: 01.03.2019
(51) Int. Cl.: A61K 9/00, A61K 47/02, A61K 47/10, A61K 47/20, A61K 47/22, A61K 47/26, A61K 47/36, A61K 47/40, A61K 31/46

(54) **OPHTHALMIC FORMULATIONS BASED ON ATROPINE**
OPHTHALMISCHE FORMULIERUNGEN AUF BASIS VON ATROPIN
FORMULATIONS OPHTALMIQUES À BASE D'ATROPINE

(43) Date of publication of application: 02.09.2020
(73) Proprietor: Medivis S.R.L., 95030 Tremestieri Eneo (CT) (IT)
(72) Inventor: ALEO, Danilo, I-95030 Tremestieri Etneo, Catania (IT); MELILLI, Barbara, I-95030 Tremestieri Etneo, Catania (IT); SAITA, Maria Grazia, I-95030 Tremestieri Etneo, Catania (IT); SPITALERI, Fabiola, I-95030 Tremestieri Etneo, Catania (IT); MANGIAFICO, Sergio, I-95030 Tremestieri Etneo, Catania (IT); CRO, Melina, I-95030 Tremestieri Etneo, Catania (IT)
(74) Representative: Croce, Valeria

(56) References cited:
- CN-A- 108 338 969
- US-A1- 2009 312 724

## Description

The present patent application discloses atropine-containing ophthalmic formulations, which are particularly stable at neutral pHs.

### Background art

Atropine is the tropine ester of tropic acid and is available on the market mainly as a sulfate salt.

Commercially available atropine sulfate formulations for ophthalmic use are formulated in acid solutions having a pH from 4 to 5.5.

In general, at these acidity levels, the formulations are not suitable and are poorly tolerated by patients; however, at these acidic pHs, atropine does not undergo hydrolysis and, therefore, can be stored at room temperature for long periods of time.

The physiological pH of the human tear is about 7.4, and an eye drop should be formulated at the same pH as the tear fluid; to date, this is however impossible for the atropine sulfate-containing eye drops since at neutral pHs atropine quickly hydrolyzes and this rate increases reducing atropine concentration (e.g., US 9,421,199), further complicating the possibility of formulating atropine at low doses.

The use of atropine at low doses (i.e., 0.01%) has become a well-established clinical treatment for decreasing the progression of myopia in children, which are the patients least tolerating an acid pH at an ocular level.

In the international patent application WO 2017/204262 formulations of atropine at 0.01% are described with various ingredients and polymers with acid pHs not exceeding 6.0; for these formulations, after 4 weeks, tropic acid values ranging from 3 to 6% are obtained which are at the limits of the desirable values, making these formulations unsuitable for a long-term storage.

The use of deuterated water (heavy water) to reduce the hydrolytic degradation of atropine in tropic acid is reported in U.S. Patent No. 9,421,199.

In practice, reducing the rate of hydrolysis through the isotopic effect is interesting only from the academic point of view given the costs of deuterated water and, above all, the *in vivo* exchange that would occur between deuterium and hydrogen in the subjects receiving these formulations.

U.S. patent application 2018/032017 discloses formulations having a pH from 5.0 to 6.0 which, stored at 25°C and at a controlled humidity of 60%, generate a quantity of tropic acid less than or equal to 0.35% after two months; however, the pH still remains far from the physiological one of 7.4.

The prior-art document CN 108 338 969 discloses an ophthalmic composition comprising atropine sulfate 0.05 (W/v), sodium hyaluronate 0.01%, NaCl 0.9%, a preservative 0.05% and betacyclodextrin 0.3%, at pH 6.2.

There is therefore a need for atropine-based formulations having pHs closer to the physiological pH of the tear fluid and which can be kept stable for long periods of time.

### Summary of the invention

The authors of the present invention have surprisingly found how to prepare atropine sulfate-based formulations in a therapeutic concentration, which are stable and with a physiological pH, and which, after 6 months, have an atropine sulfate content greater than 99.5% and a tropic acid content less than 0.5%.

### Subject of invention

The present patent application first relates to atropine sulfate-comprising ophthalmic compositions as defined in the claims.

In a second object, the ophthalmic compositions of the invention are disclosed for medical use.

In another object, the ophthalmic compositions of the invention are disclosed for pediatric medical use.

In a still further object, a method is disclosed for the stabilization of atropine-comprising ophthalmic compositions, or a pharmaceutically acceptable salt thereof.

### Description of the invention

According to a first object, the present invention relates to atropine-based ophthalmic compositions.

In particular, the atropine is in the form of a sulfate salt, or another pharmaceutically acceptable salt.

For the purposes of the present invention, atropine, or a pharmaceutically acceptable salt thereof, is present in the compositions of the invention in an amount of about 0.001-2.0% (w/w).

In one aspect of the invention, the described compositions further comprise a buffering agent.

Such buffering agent is preferably selected from the group comprising: sodium and/or potassium salts of phosphoric acid, or borate buffer.

The buffering agent can be included in an amount such as to maintain the pH of the ophthalmic composition from 6.0 to 7.2.

According to another aspect of the invention, the described compositions comprise an osmotizing agent.

This osmotizing agent is preferably selected from the group comprising: glycerol, trehalose, inosine phosphate, or combinations thereof, or salts, e.g. sodium chloride, potassium chloride, and magnesium chloride.

The osmotizing agent can be included in an amount of about 0.2-4% (w/w).

According to a further aspect of the invention, the described compositions comprise viscosifying agents.

The viscosifying agent can be included in an amount of about 0.05-2% (w/w).

These viscosifying agents are selected within the viscosifying agents for ophthalmic use.

In a preferred aspect, this agent is represented by hyaluronic acid, or a pharmaceutically acceptable salt thereof, e.g. sodium hyaluronate.

Preferably, the hyaluronic acid which can be used for the purposes of the present invention is characterized by a molecular weight from about 900 to 4,000 KDa.

When present as a viscosifying agent, hyaluronic acid is present in an amount of about 0.01-0.25% (w/w).

As per the present invention, the described compositions comprise sulfobutyl ether β-cyclodextrin (SBECD).

Such cyclodextrin can be included in an amount of about 0.2-20% (w/w).

In particular, where the atropine concentrations are of 0.01% (w/w), the sulfobutyl ether β-cyclodextrin (SBECD) can be present in an amount of about 0.2-4%, and preferably of about 2% (w/w).

According to a preferred aspect of the present invention, the atropine sulfate, or a pharmaceutically acceptable salt thereof, is included in an amount of about 0.001-2.0%, and the sulfobutyl ether β-cyclodextrin is included in an amount of about 0.5-20% (w/w).

According to a more preferred aspect, the atropine sulfate, or a pharmaceutically acceptable salt thereof, is present in an amount of about 0.08-0.012%, and the sulfobutyl ether β-cyclodextrin is included in an amount of about 0.5% to 4.0% (w/w).

According to an even more preferred aspect, the atropine sulfate, or a pharmaceutically acceptable salt thereof, is present in an amount of about 0.45-0.55%, and the sulfobutyl ether β-cyclodextrin is included in an amount of about 6-12% (w/w).

According to another preferred aspect, the atropine sulfate, or a pharmaceutically acceptable salt thereof, is present in an amount of about 0.8-1.2%, and the sulfobutyl ether β-cyclodextrin is included in an amount of about 10-18% (w/w).

The compositions of the invention also comprise water for injectables.

The compositions described in the present patent application, due to their particular formulation, have advantageously shown to be stable at a room temperature of 25°C, for at least 6 months.

In particular, this stability is at 60% relative humidity conditions.

When so stored, the compositions have been shown to maintain an atropine sulphate content greater than about 99.5%.

In a preferred aspect of the invention, the compositions of the invention have shown, after having been stored under the above-indicated conditions after 6 months, to give rise to a tropic acid content, formed by the degradation, which is less than about 0.5%.

According to a second object, the ophthalmic compositions of the invention are described for medical use.

According to another aspect of the present invention, the ophthalmic compositions of the present invention are described for pediatric medical use.

Advantageously, this use is possible in children even under the age of 16, or even under the age of 10.

More specifically, this medical use is to decrease the progression of myopia in the child.

According to a further object, a method is described for the stabilization of atropine-comprising ophthalmic compositions, or a pharmaceutically acceptable salt thereof.

This stabilization, in particular, is obtained by the addition of sulfobutyl ether β-cyclodextrin in the above-described compositions.

Stabilization is linked to the pH of the composition, which must be from 6.0 to 7.2.

The stabilization effect is shown up to 6 months, when the compositions are stored at room temperature (25°C) and at a relative humidity of 60%.

Below are some examples of formulations according to the above description.

### EXAMPLE 1

### Formulations F1-F5

The following table shows examples of compositions according to the invention having the formulations F1 to F5.

| **Ingredients** | **% w/w F1** | **% w/w F2** | **% w/w F3** | **% w/w F4** | **% w/w F5** |
|---|---|---|---|---|---|
| Atropine sulfate | 0.008 | 0.008 | 0.01 | 0.01 | 0.01 |
| Sulfobutyl ether β-cyclodextrin | 1 | 2 | 1.5 | 2 | 2.5 |
| Sodium Hyaluronate (HA) | 0.05 | 0.05 | 0.2 | 0.05 | 0.1 |
| Inosine 5' phosphate | 0.1 | 0.1 | 0.1 | 0.1 | 0.1 |
| MgCl₂ * 6H₂O | 0.15 | 0.15 | 0.15 | 0.15 | 0.15 |
| KCl | 0.15 | 0.15 | 0.15 | 0.15 | 0.15 |
| Glycerol | 0.7 | 0.7 | 0.7 | 0.7 | 0.7 |
| Na₂HPO₄*12 H₂O | 0.2 | 0.2 | 0.2 | 0.2 | 0.2 |
| NaH₂PO₄*2 H₂O | 0.065 | 0.065 | 0.065 | 0.065 | 0.065 |
| Trehalose | 2 | 2 | 2 | 2 | 2 |
| Water for injectables | Up to 100 gr | Up to 100 gr | Up to 100 gr | Up to 100 gr | Up to 100 gr |
| pH | 6.5 | 6.8 | 6.5 | 6.8 | 7.0 |

### EXAMPLE 2

### Formulation F6-F9

The following table shows examples of compositions according to the invention having the formulations F6 to F9.

| **Ingredients** | **% w/w F6** | **% w/w F7** | **% w/w F8** | **% w/w F9** |
|---|---|---|---|---|
| Atropine sulfate | 0.5 | 0.5 | 1 | 1 |
| Sulfobutyl ether β-cyclodextrin | 6 | 10 | 10 | 16 |
| Sodium Hyaluronate (HA) | 0.05 | 0.05 | 0.05 | 0.05 |
| Inosine 5' phosphate | 0.1 | 0.1 | -- | -- |
| MgCl₂ * 6H₂O | 0.15 | -- | -- | -- |
| KCl | -- | -- | -- | -- |
| Na₂HPO₄*12 H₂O | 0.2 | 0.2 | 0.2 | 0.2 |
| NaH₂PO₄*2 H₂O | 0.065 | 0.065 | 0.065 | 0.065 |

| | | | | |
|---|---|---|---|---|
| Water for injectables | Up to 100 gr | Up to 100 gr | Up to 100 gr | Up to 100 gr |
| pH | 6.5 | 6.8 | 6.5 | 6.8 |

The formulations of the examples have all proved to be stable and to maintain, after storage at room temperature of 25°C, for 6 months, at a relative humidity of 60%, an atropine sulphate content greater than 99.5%, and a tropic acid content, formed by degradation, less than 0.5%.

From the above description, the advantages offered by the ophthalmic compositions subject of the present invention will be evident to those skilled in the art.

In particular, the formulation herein provided allows to extend the expiry of the product, as it can be stored at room temperature for a full 6 months while maintaining a sufficient atropine content for therapeutic purposes.

Moreover, the described formulations allow to prepare compositions with a low content of atropine suitable for pediatric application.

## Claims

1. An ophthalmic composition comprising atropine sulfate, or a pharmaceutically acceptable salt thereof, and sulfobutyl ether β-cyclodextrin (SBECD), **characterized by** a pH from 6.0 to 7.2.

2. An ophthalmic composition according to the preceding claim, wherein said atropine sulfate, or a pharmaceutically acceptable salt thereof, is present in an amount of about 0.001-2.0% (w/w).

3. An ophthalmic composition according to claim 1 or 2, wherein said sulfobutyl ether β-cyclodextrin is included in an amount of about 0.2-20% (w/w).

4. An ophthalmic composition according to any one of the preceding claims, wherein atropine sulfate, or pharmaceutically acceptable salts thereof, are included in an amount of about 0.001-2.0%, and sulfobutyl ether β-cyclodextrin is included in an amount of about 0.5-20% (w/w).

5. An ophthalmic composition according to any one of the preceding claims, wherein atropine sulfate, or a pharmaceutically acceptable salt thereof, is present in an amount of about 0,08-0,0120, and sulfobutyl ether β-cyclodextrin is included in an amount of about 0.5% to 4.0% (w/w).

6. An ophthalmic composition according to any one of the preceding claims 1 to 4, wherein atropine sulfate, or a pharmaceutically acceptable salt thereof, is present in an amount of about 0.45-0.55%, and sulfobutyl ether β-cyclodextrin is included in an amount of about 6-12% (w/w).

7. An ophthalmic composition according to any one of the preceding claims 1 to 4, wherein atropine sulfate, or a pharmaceutically acceptable salt thereof, is present in an amount of about 0.8-1.2%, and sulfobutyl ether β-cyclodextrin is included in an amount of about 10-18% (w/w).

8. An ophthalmic composition according to any one of the preceding claims 1 to 3, wherein atropine sulfate, or a pharmaceutically acceptable salt thereof, is present in an amount of about 0.01% (w/w), and sulfobutyl ether β-cyclodextrin is included in an amount of about 0.2-4%, and preferably of about 2% (w/w).

9. An ophthalmic composition according to any one of the preceding claims further comprising a viscosifying agent and an osmotizing agent.

10. An ophthalmic composition according to any one of the preceding claims 1 to 4 having one of the following formulations:
| **Ingredients** | **% w/w F1** | **% w/w F2** | **% w/w F3** | **% w/w F4** | **% w/w F5** |
|---|---|---|---|---|---|
| Atropine sulfate | 0.008 | 0.008 | 0.01 | 0.01 | 0.01 |
| Sulfobutyl ether β-cyclodextrin | 1 | 2 | 1.5 | 2 | 2.5 |
| Sodium Hyaluronate (HA) | 0.05 | 0.05 | 0.2 | 0.05 | 0.1 |
| Inosine 5' phosphate | 0.1 | 0.1 | 0.1 | 0.1 | 0.1 |
| MgCl₂ * 6H₂O | 0.15 | 0.15 | 0.15 | 0.15 | 0.15 |
| KCl | 0.15 | 0.15 | 0.15 | 0.15 | 0.15 |
| Glycerol | 0.7 | 0.7 | 0.7 | 0.7 | 0.7 |
| Na₂HPO₄*12 H₂O | 0.2 | 0.2 | 0.2 | 0.2 | 0.2 |
| NaH₂PO₄*2 H₂O | 0.065 | 0.065 | 0.065 | 0.065 | 0.065 |
| Trehalose | 2 | 2 | 2 | 2 | 2 |
| Water for injectables | Up to 100 gr | Up to 100 gr | Up to 100 gr | Up to 100 gr | Up to 100 gr |
| pH | 6.5 | 6.8 | 6.5 | 6.8 | 7.0 |
or
| **Ingredients** | **% w/w F6** | **% w/w F7** | **% w/w F8** | **% w/w F9** |
|---|---|---|---|---|
| Atropine sulfate | 0.5 | 0.5 | 1 | 1 |
| SBECD | 6 | 10 | 10 | 16 |
| Sodium Hyaluronate (HA) | 0.05 | 0.05 | 0.05 | 0.05 |
| Inosine 5' phosphate | 0.1 | 0.1 | -- | -- |
| MgCl₂ * 6H₂O | 0.15 | -- | -- | -- |
| KCl | -- | -- | -- | -- |
| Na₂HPO₄*12 H₂O | 0.2 | 0.2 | 0.2 | 0.2 |
| NaH₂PO₄*2 H₂O | 0.065 | 0.065 | 0.065 | 0.065 |
| Water for injectables | Up to 100 gr | Up to 100 gr | Up to 100 gr | Up to 100 gr |
| pH | 6.5 | 6.8 | 6.5 | 6.8 |

11. An ophthalmic composition according to any one of the preceding claims for medical use.

12. An ophthalmic composition according to claim 8 for medical use to decrease the progression of myopia in children.

## Patentansprüche

1. Ophthalmische Zusammensetzung, umfassend Atropinsulfat, oder ein pharmazeutisch akzeptables Salz davon und Sulfobutylether-β-cyclodextrin (SBECD), **gekennzeichnet durch** eine pH von 6,0 bis 7,2.

2. Ophthalmische Zusammensetzung nach dem vorhergehenden Anspruch, wobei das Atropinsulfat, oder ein pharmazeutisch akzeptables Salz davon in einer Menge von etwa 0,001-2,0% (Gewicht/Gewicht) vorhanden ist.

3. Ophthalmische Zusammensetzung nach Anspruch 1 oder 2, wobei das Sulfobutylether-β-cyclodextrin in einer Menge von etwa 0,2-20% (Gewicht/Gewicht) umfasst ist.

4. Ophthalmische Zusammensetzung nach einem der vorhergehenden Ansprüche, wobei Atropinsulfat, oder pharmazeutisch akzeptable Salze davon in einer Menge von etwa 0,001-2,0% umfasst sind und Sulfobutylether-β-cyclodextrin in einer Menge von etwa 0,5-20% (Gewicht/Gewicht) umfasst ist.

5. Ophthalmische Zusammensetzung nach einem der vorhergehenden Ansprüche, wobei Atropinsulfat, oder ein pharmazeutisch akzeptables Salz davon in einer Menge von etwa 0,08-0,012% vorhanden ist und Sulfobutylether-β-cyclodextrin in einer Menge von etwa 0,5% bis 4,0% (Gewicht/Gewicht) umfasst ist.

6. Ophthalmische Zusammensetzung nach einem der vorhergehenden Ansprüche 1 bis 4, wobei Atropinsulfat, oder ein pharmazeutisch akzeptables Salz davon in einer Menge von etwa 0,45-0,55% vorhanden ist und Sulfobutylether-β-cyclodextrin in einer Menge von etwa 6-12% (Gewicht/Gewicht) umfasst ist.

7. Ophthalmische Zusammensetzung nach einem der vorhergehenden Ansprüche 1 bis 4, wobei Atropinsulfat, oder ein pharmazeutisch akzeptables Salz davon in einer Menge von etwa 0,8-1,2% vorhanden ist und Sulfobutylether-β-cyclodextrin in einer Menge von etwa 10-18% (Gewicht/Gewicht) umfasst ist.

8. Ophthalmische Zusammensetzung nach einem der vorhergehenden Ansprüche 1 bis 3, wobei Atropinsulfat, oder ein pharmazeutisch akzeptables Salz davon in einer Menge von etwa 0,01% (Gewicht/Gewicht) vorhanden ist und Sulfobutylether-β-cyclodextrin in einer Menge von etwa 0,2-4%, und vorzugsweise von etwa 2% (Gewicht/Gewicht) umfasst ist.

9. Ophthalmische Zusammensetzung nach einem der vorhergehenden Ansprüche, ferner umfassend ein viskosifizierendes Agens und ein osmotisierendes Agens.

10. Ophthalmische Zusammensetzung nach einem der vorhergehenden Ansprüche 1 bis 4 mit einer der folgenden Formulierungen:
| **Inhaltsstoffe** | **% Gew./Gew. F1** | **% Gew./Gew. F2** | **% Gew./Gew. F3** | **% Gew./Gew. F4** | **% Gew./Gew. F5** |
|---|---|---|---|---|---|
| Atropinsulfat | 0,008 | 0,008 | 0,01 | 0,01 | 0,01 |
| Sulfobutylether-β-cyclodextrin | 1 | 2 | 1,5 | 2 | 2,5 |
| Natriumhyaluronat (HA) | 0,05 | 0,05 | 0,2 | 0,05 | 0,1 |
| Inosin-5'-phosphat | 0,1 | 0,1 | 0,1 | 0,1 | 0,1 |
| MgCl₂ - 6H₂O | 0,15 | 0,15 | 0,15 | 0,15 | 0,15 |
| KCI | 0,15 | 0,15 | 0,15 | 0,15 | 0,15 |
| Glycerin | 0,7 | 0,7 | 0,7 | 0,7 | 0,7 |
| Na₂HPO₄ - 12H₂O | 0,2 | 0,2 | 0,2 | 0,2 | 0,2 |
| NaH₂PO₄ - 2H₂O | 0,065 | 0,065 | 0,065 | 0,065 | 0,065 |
| Trehalose | 2 | 2 | 2 | 2 | 2 |
| Wasser für Injektionsmittel | Bis zu 100 gr | Bis zu 100 gr | Bis zu 100 gr | Bis zu 100 gr | Bis zu 100 gr |
| pH | 6,5 | 6,8 | 6,5 | 6,8 | 7,0 |
| **Inhaltsstoffe** | **% Gew./Gew. F6** | **% Gew./Gew. F7** | **% Gew./Gew. F8** | **% Gew./Gew. F9** |
|---|---|---|---|---|
| Atropinsulfat | 0,5 | 0,5 | 1 | 1 |
| SBECD | 6 | 10 | 10 | 16 |
| Natriumhyaluronat (HA) | 0,05 | 0,05 | 0,05 | 0,05 |
| Inosin-5'-phosphat | 0,1 | 0,1 | - | - |
| MgCl₂ - 6H₂O | 0,15 | - | - | - |
| KCl | - | - | - | - |
| Na₂HPO₄ - 12H₂O | 0,2 | 0,2 | 0,2 | 0,2 |
| NaH₂PO₄ - 2H₂O | 0,065 | 0,065 | 0,065 | 0,065 |
| Wasser für Injektionsmittel | Bis zu 100 gr | Bis zu 100 gr | Bis zu 100 gr | Bis zu 100 gr |
| pH | 6,5 | 6,8 | 6,5 | 6,8 |

11. Ophthalmische Zusammensetzung nach einem der vorhergehenden Ansprüche zur medizinischen Verwendung.

12. Ophthalmische Zusammensetzung nach Anspruch 8 zur medizinischen Verwendung zur Verringerung des Fortschreitens von Myopie bei Kindern.

## Revendications

1. Composition ophtalmique comprenant du sulfate d'atropine, ou un sel pharmaceutiquement acceptable de celui-ci, et de la sulfobutyléther-β-cyclodextrine (SBECD), **caractérisée par** un pH de 6,0 à 7,2.

2. Composition ophtalmique selon la revendication précédente, dans laquelle ledit sulfate d'atropine, ou un sel pharmaceutiquement acceptable de celui-ci, est présent dans une quantité d'environ 0,001 à 2,0 % en poids.

3. Composition ophtalmique selon la revendication 1 ou 2, dans laquelle ladite sulfobutyléther-β-cyclodextrine est incluse dans une quantité d'environ 0,2 à 20 % en poids.

4. Composition ophtalmique selon l'une quelconque des revendications précédentes, dans laquelle le sulfate d'atropine, ou un sel pharmaceutiquement acceptable de celui-ci, est inclus dans une quantité d'environ 0,001à 2,0 %, et la sulfobutyléther-β-cyclodextrine est incluse dans une quantité d'environ 0,5 à 20 % en poids.

5. Composition ophtalmique selon l'une quelconque des revendications précédentes, dans laquelle le sulfate d'atropine, ou un sel pharmaceutiquement acceptable de celui-ci, est présent dans une quantité d'environ 0,08 à 0,012 %, et la sulfobutyléther-β-cyclodextrine est incluse dans une quantité d'environ 0,5 % à 4,0 % en poids.

6. Composition ophtalmique selon l'une quelconque des revendications 1 à 4 précédentes, dans laquelle le sulfate d'atropine, ou un sel pharmaceutiquement acceptable de celui-ci, est présent dans une quantité d'environ 0,45 à 0,55 %, et la sulfobutyléther-β-cyclodextrine est incluse dans une quantité d'environ 6 à 12 % en poids.

7. Composition ophtalmique selon l'une quelconque des revendications 1 à 4 précédentes, dans laquelle le sulfate d'atropine, ou un sel pharmaceutiquement acceptable de celui-ci, est présent dans une quantité d'environ 0,8 à 1,2 %, et la sulfobutyléther-β-cyclodextrine est incluse dans une quantité d'environ 10 à 18 % en poids.

8. Composition ophtalmique selon l'une quelconque des revendications 1 à 3 précédentes, dans laquelle le sulfate d'atropine, ou un sel pharmaceutiquement acceptable de celui-ci, est présent dans une quantité d'environ 0,01 % en poids, et la sulfobutyléther-β-cyclodextrine est incluse dans une quantité d'environ 0,2 à 4 %, et est de préférence d'environ 2 % en poids.

9. Composition ophtalmique selon l'une quelconque des revendications précédentes, comprenant en outre un agent améliorant la viscosité et un agent d'osmotisation.

10. Composition ophtalmique selon l'une quelconque des revendications 1 à 4 précédentes, présentant l'une des formulations suivantes :
| **Ingrédient** | **% en poids F1** | **% en poids F2** | **% en poids F3** | **% en poids F4** | **% en poids F5** |
|---|---|---|---|---|---|
| Sulfate d'atropine | 0,008 | 0,008 | 0,01 | 0,01 | 0,01 |
| Sulfobutyléther-β-cyclodextrine | 1 | 2 | 1,5 | 2 | 2,5 |
| Hyaluronate de sodium (HA) | 0,05 | 0,05 | 0,2 | 0,05 | 0,1 |
| Inosine-5'-phosphate | 0,1 | 0,1 | 0,1 | 0,1 | 0,1 |
| MgCl₂ * 6 H₂O | 0,15 | 0,15 | 0,15 | 0,15 | 0,15 |
| KCI | 0,15 | 0,15 | 0,15 | 0,15 | 0,15 |
| Glycérol | 0,7 | 0,7 | 0,7 | 0,7 | 0,7 |
| Na₂HPO₄*12 H₂O | 0,2 | 0,2 | 0,2 | 0,2 | 0,2 |
| NaH₂PO₄*2 H₂O | 0,065 | 0,065 | 0,065 | 0,065 | 0,065 |
| Tréhalose | 2 | 2 | 2 | 2 | 2 |
| Eau pour préparation injectable | jusqu'à 100 g | jusqu'à 100 g | jusqu'à 100 g | jusqu'à 100 g | jusqu'à 100 g |
| pH | 6,5 | 6,8 | 6,5 | 6,8 | 7,0 |
ou
| **Ingrédient** | **% en poids F6** | **% en poids F7** | **% en poids F8** | **% en poids F9** |
|---|---|---|---|---|
| Sulfate d'atropine | 0,5 | 0,5 | 1 | 1 |
| SBECD | 6 | 10 | 10 | 16 |
| Hyaluronate de sodium (HA) | 0,05 | 0,05 | 0,05 | 0,05 |
| Inosine-5'-phosphate | 0,1 | 0,1 | -- | -- |
| MgCl₂ * 6 H₂O | 0,15 | -- | -- | -- |
| KCl | -- | -- | -- | -- |
| Na₂HPO₄^{∗}12 H₂O | 0,2 | 0,2 | 0,2 | 0,2 |
| NaH₂PO₄*2 H₂O | 0,065 | 0,065 | 0,065 | 0,065 |
| Eau pour préparation injectable | jusqu'à 100 g | jusqu'à 100 g | jusqu'à 100 g | jusqu'à 100 g |
| pH | 6,5 | 6,8 | 6,5 | 6,8 |

11. Composition ophtalmique selon l'une quelconque des revendications précédentes, destinée à un usage médical.

12. Composition ophtalmique selon la revendication 8, destinée à un usage médical visant à ralentir la progression de la myopie chez l'enfant.
